# EUROPEAN PATENT SPECIFICATION

(11) **EP 0 638 319 B1**
(45) Date of publication and mention of the grant of the patent: **19.01.2000**
(21) Application number: 94305778.6
(22) Date of filing: 04.08.1994
(51) Int. Cl.: A61L 2/04

(54) **Apparatus and method for sterilizing fine powders**
Vorrichtung und Verfahren zur Sterilisierung von feinen Pulvern
Appareil et procédé pour la stérilisation de poudres fines

(30) Priority: 04.08.1993 GB 9316177
(43) Date of publication of application: 15.02.1995
(73) Proprietor: COLIN STEWART MINCHEM LTD., Winsford, Cheshire CW7 3BU (GB)
(72) Inventor: Kitching, Peter Jeffrey, Winsford, Cheshire (GB)
(74) Representative: Chettle, Adrian John

(56) References cited:
- DE-A- 2 128 805
- FR-A- 2 331 355

## Description

This invention relates to an apparatus and method for sterilizing fine powders such as talcum powder.

Talcum powder for personal use requires sterilization to kill potentially harmful organisms. One prior art sterilization method comprises placing a bulk quantity of talcum powder in a vacuum chamber, evacuating the air, and subsequently flooding the chamber with ethylene dioxide. This method is effective but has the disadvantage that under certain conditions ethylene dioxide may be explosive; to counter this threat it has become common to mix a quantity of CFC's (chloro-fluoro carbons) with the ethylene dioxide. Current legislation to limit the use of CFC's is rendering this method of sterilization less desirable, and may eventually force manufacturers and processors to cease using the method.

Radiation might as an alternative be used to sterilize talcum powder but this method is rather expensive, and requires close control of the process and skilled staff.

Another method of sterilization is to use heat, but a heat process must be designed to ensure that no relatively cool pockets of unsterilized material remain, for otherwise the process may serve only to encourage multiplication of dangerous organisms. Furthermore the temperature must not be so great as to cause burning or degradation of the powder.

It has been suggested to use an air conveyor to carry powder through a duct sufficiently hot to give effective sterilization. However an airspeed of around fifteen metres per second is required to carry the powder, and this results in an excessively long duct if temperatures are to be kept at a reasonable level. The shorter the duct, the higher the temperature required. In any event sterilization times are measured in seconds rather than minutes, thus requiring very close control of the process if effective sterilization without powder degradation is to be achieved.

The apparatus and method of the present invention provides for heat sterilization in an effective manner with a relatively long residence time and without the risk of powder degradation at excessive temperatures.

According to one aspect of the invention there is provided apparatus for sterilizing a fine powder and comprising a tubular sterilizing duct, means for heating the duct to a sterilizing temperature, means for introducing fine powder at one end of the duct and for collecting fine powder from the other end of the duct, and air introduction means at one end of the duct for fluidising powder in said duct.

In a preferred embodiment the heating means comprises a jacket encircling the duct and capable of heating the duct to above a temperature of 180°C.

In another aspect, the invention provides a method of sterilizing fine powders in the aforesaid apparatus and comprising the steps of
a) heating the sterilizing duct to a temperature of approximately 500°C;
b) introducing fine powder at one end of the duct and removing fine powder from the other end of the duct with a powder residence time in the duct sufficient to heat the powder to above 120°C;
c) introducing air into the duct at a rate of 30-60 litres per minute.

Preferably the duct is a substantially vertical tube through which fine powder passes downwardly, air being introduced at the base of the tube and passing upwardly to fluidise the powder. The tube preferably has a diameter in the range 75-150 mm and a height in the range 4-5 metres.

The apparatus may further include a hopper at the outlet end of said duct to collect hot powder, and optionally a cooler to cool the powder for discharge and further processing.

The hopper may be insulated to permit the powder to soak at an elevated temperature. After leaving the hopper, the powder may enter a cooler which may comprise a substantially vertical tube through which the powder passes from top to bottom, and having an external water jacket through which coolant is passed in use.

In a preferred embodiment a sterilizing tube and a cooling tube are upright and arranged adjacent one another, the hopper being above the cooling tube and lift means being provided to transfer powder from the base of the sterilizing tube to the hopper.

The method may include the further steps of
d) permitting the fine powder to soak at an elevated temperature for at least 15 minutes on exit from the sterilizing duct; and
e) cooling the fine powder for discharge and further processing.

Other aspects of the invention will be apparent from the following description of a preferred embodiment illustrated by way of example in the accompanying diagrammatic sketch of sterilization apparatus for talcum powder.

Unsterilized talcum powder is conveyed from silos into a feed hopper (11) in any suitable manner and indicated by arrow A - the feed hopper is typically topped-up periodically on signal from a device sensing low powder level.

Powder is transferred from hopper 11 at a controlled rate, for example by-feed screw 12, to the upper end of a substantially upright sterilizing tube 13. The sterilizing tube 13 has a heating jacket of any suitable type, for example containing electrical elements, and is insulated.

The base of the sterilizing tube 13 is closed by an end plate 14 having an upwardly facing nozzle therein and connections (not shown) to a source of air under pressure. The direction of air flow is indicated by arrow B.

Powder passes from a point just above the base of the sterilizing tube 13 (arrow C) to a discharge controller and elevator 15 of any suitable type, and from the elevator 15 (arrow D) to an insulated hopper 16.

The hopper 16 discharges into a substantially upright cooling tube 17 surrounded by a water jacket through which coolant is passed in a counterflow direction. The base of the cooling tube 17 is closed by a second end plate 18 also having a nozzle for connection to a source of air under pressure. The direction of airflow is indicated by arrow E.

Powder passes from a point just above the base of the cooling tube 17 (arrow F) to a discharge controller and elevator 19 of any suitable type, and from the elevator 19 (arrow G) to a storage bin 20.

In operation the sterilizing tube is maintained substantially full with a powder residence time of about 20 minutes and a discharge temperature of around 220°C for a tube of 100 mm internal diameter and a height of 4.5 metres. The residence time is determined by the discharge controller and elevator 15, and may be varied to suit actual powder temperatures achieved. Powder temperature is monitored by temperature sensors of any suitable kind and it is envisaged that the sterilization process may be automated such that residence time is a minimum for given powder temperatures.

Air is bubbled through the sterilizing tube 13 and rises through the powder for discharge at the top. The air fluidises the bed and promotes heat transfer and free particle movement. The rate of air injection, and the injection pressure are not of prime importance to the process provided that they are sufficient to fluidise the column of powder. For a tube of the stated dimensions and talcum powder of 0.074 mm (200 mesh size), an air flow rate of 45 litres per minute at an injection pressure of 2 bar has been found satisfactory.

The feed screw 12 tops-up the sterilizing tube 13 under the control of level sensors in the tube 13, or in accordance with the volume drawn off by the controller and elevator 15.

The insulated hopper 16 is provided to give a heat soak of around 20 minutes, thus ensuring that the hopper and the contents are fully sterilized.

Once sterilised the powder is cooled for discharge and transport. A jacketed cooling tube of any suitable kind may be provided, and the powder may optionally be fluidised to promote even cooling and free powder movement.

The rate of powder transfer through the cooling tube is determined by the discharge controller and elevator 19.

The storage bins 20 may be of a size and type suitable for transport by road, or alternatively the powder may be discharged into a tanker or silo.

A bank of sterilizing tubes may be arranged to feed a single insulated hopper 16, thus retaining the beneficial length/diameter ratio of the sterilizing tube within limits which can be installed in a conventional industrial building, and permitting only a proportion of the sterilizing tubes to be operated at times when demand is low or maintenance is required.

The hot powder sterilizes those components of the process with which it comes into contact, and thus special attention is only required for equipment downstream of the insulated hopper 16. Air introduced into the sterilizing tube need not be itself sterilized but should be filtered. Air introduced to the cooling tube must be clean and sterile.

In certain circumstances, especially for batch production, the cooling tube may not be required, and the insulated hopper 16 may alternatively be replaced by a conventional sealed silo or sterile tank.

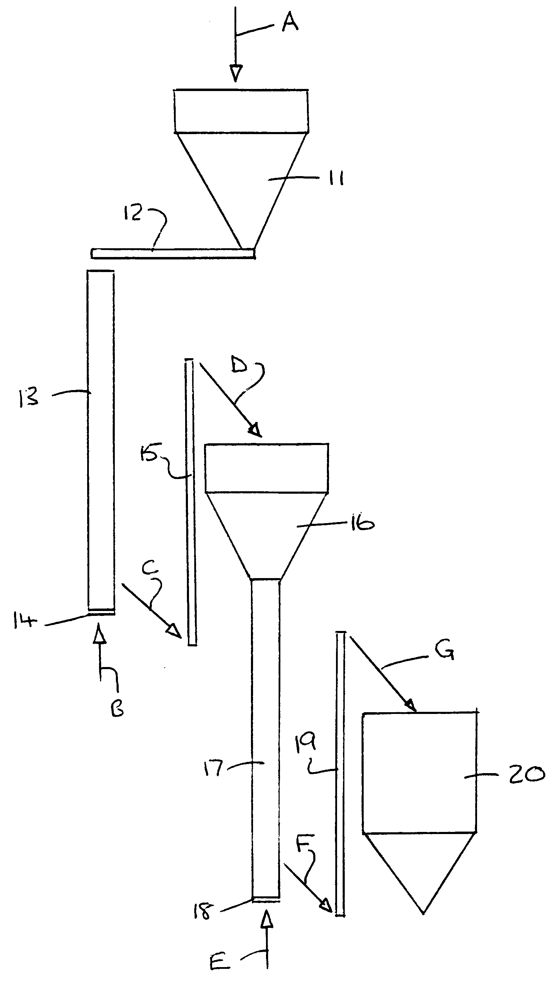

## Claims

1. Apparatus for sterilising a fine powder and comprising a tubular sterilising duct (13), means for heating the duct to a sterilising temperature, means (11,12) for introducing fine powder at one end of the duct and for collecting the fine powder from the other end of the duct, and air introduction means at one end of the duct for fluidising the fine powder in said duct.

2. Apparatus according to Claim 1, wherein the heating means comprises a jacket encircling the duct and capable of heating the duct to above a temperature of 180°C.

3. Apparatus according to Claim 1 or Claim 2, wherein the duct is a substantially vertical tube through which fine powder passes downwardly, air being introduced at the base of the tube and passing upwardly to fluidise the powder.

4. Apparatus according to Claim 3, wherein the tube has a diameter in the range of 75-150 mm and a height in the range of 4-5 metres.

5. Apparatus according to any preceding claim and including a hopper (16) at the outlet end of said duct to collect hot powder.

6. Apparatus according to Claim 5, wherein the hopper is insulated.

7. Apparatus according to Claim 5 or Claim 6 and further including a cooler (17) downstream of said hopper.

8. Apparatus according to Claim 7, wherein the cooler (17) comprises a substantially vertical tube through which the powder passes from top to bottom, and an external water jacket through which coolant is passed in use.

9. Apparatus according to Claim 7 or Claim 8, wherein the sterilising tube and the cooling tube are upright and arranged adjacent one another, the hopper (16) being above the cooling tube (17) and lift means (15) being provided to transfer powder from the base of the sterilising tube to the hopper.

10. A method of sterilising fine powders and comprising the steps of:
a) heating a sterilizing duct to a temperature of approximately 500°C;
b) introducing fine powder at one end of the duct and removing fine powder from the other end of the duct with a powder residence time in the duct sufficient to heat the powder to above 120°C;
c) introducing air into the duct at a rate of 30-60 litres per minute.

11. A method of sterilizing fine powders according to Claim 10 and further including the steps of:
d) permitting the fine powder to soak at an elevated temperature for at least 15 minutes on exit from the sterilizing duct; and
e) cooling the fine powder for discharge and further processing.

## Patentansprüche

1. Vorrichtung zum Sterilisieren eines feinen Pulvers und mit einer rohrförmigen Sterilisierungsleitung (13), mit einem Mittel zum Heizen der Leitung auf eine Sterilisationstemperatur, mit einem Mittel (11, 12) zum Einleiten eines feinen Pulvers an einem Ende der Leitung und zum Sammeln des feinen Pulvers von dem anderen Ende der Leitung, und mit einem Lufteinleitungsmittel an einem Ende der Leitung zum Fluidisieren des feinen Pulvers in der Leitung.

2. Vorrichtung nach Anspruch 1, wobei das Heizmittel eine Buchse aufweist, die die Leitung umgibt und die in der Lage ist, die Leitung auf eine Temperatur von mehr als 180°C zu erwärmen.

3. Vorrichtung nach Anspruch 1 oder 2, wobei die Leitung ein im wesentlichen vertikales Rohr ist, durch das das feine Pulver nach unten tritt, wobei Luft an der Basis des Rohrs eingeleitet wird und nach oben strömt, um das Pulver zu fluidisieren.

4. Vorrichtung nach Anspruch 3, wobei das Rohr einen Durchmesser im Bereich von 75 - 150mm und eine Höhe im Bereich von 4 - 5m hat.

5. Vorrichtung nach einem der vorhergehenden Ansprüche und mit einem Aufgabetrichter (16) am Auslaßende der Leitung zum Sammeln des heißen Pulvers.

6. Vorrichtung nach Anspruch 5, wobei der Aufgabetrichter isoliert ist.

7. Vorrichtung nach Anspruch 5 oder 6, ferner mit einem Kühler (17) stromabwärts von dem Aufgabetrichter.

8. Vorrichtung nach Anspruch 7, wobei der Kühler (17) ein im wesentlichen vertikales Rohr umfaßt, durch das das Pulver von der Spitze bis zum Boden hindurchtritt, und mit einer äußeren Wasserbuchse, durch die im Betrieb Kühlmittel geleitet wird.

9. Vorrichtung nach Anspruch 7 oder 8, wobei das Sterilisationsrohr und das Kühlrohr aufrecht stehend und benachbart zueinander angeordnet sind, wobei der Aufgabetrichter (16) sich über dem Kühlrohr (17) befindet, und wobei ein Hebemittel (15) vorgesehen ist, um das Pulver von der Basis des Sterilisationsrohrs zu dem Aufgabetrichter zu übertragen.

10. Verfahren zum Sterilisieren eines feinen Pulvers mit den Schritten:
a) Heizen einer Sterilisationsleitung auf eine Temperatur von etwa 500°C;
b) Einleiten eines feinen Pulvers an einem Ende der Leitung und Entfernen des feinen Pulvers von dem anderen Ende der Leitung mit einer Pulververweilzeit in der Leitung, die ausreicht, um das Pulver mehr als 120°C zu erwärmen;
c) Einleiten von Luft in die Leitung mit einer Rate von 30-60 Litern/Minute.

11. Verfahren zum Sterilisieren von feinen Pulvern nach Anspruch 10 und ferner mit den Schritten:
d) Das feine Pulver bei einer erhöhten Temperatur mindestens 15 Minuten beim Austritt aus der Sterilisationsleitung verweilen zu lassen; und
e) Kühlen des feinen Pulvers zum Ausstoß und zu weiterer Verarbeitung.

## Revendications

1. Appareil pour la stérilisation d'une poudre fine comprenant un conduit tubulaire de stérilisation (13), un moyen de chauffage du conduit jusqu'à une température de stérilisation, des moyens (11, 12) d'introduction d'une poudre fine à une extrémité du conduit et de récupération de la poudre fine à l'autre extrémité du conduit et un moyen d'introduction d'air à l'une des extrémités du conduit pour fluidiser la poudre fine dans ledit conduit.

2. Appareil selon la revendication 1, dans lequel le moyen de chauffage comprend une enveloppe entourant le conduit, susceptible de chauffer le conduit jusqu'à une température supérieure à 180°C.

3. Appareil selon la revendication 1 ou la revendication 2, dans lequel le conduit est un tube sensiblement vertical le long duquel descend une poudre fine, de l'air étant introduit à la base du tube et le traverse de bas en haut afin de fluidiser la poudre.

4. Appareil selon la revendication 3, dans lequel le tube présente un diamètre compris entre 75 et 150 mm et une hauteur comprise entre 4 et 5 mètres.

5. Appareil selon l'une quelconque des revendications précédentes, comportant en outre une trémie (16) à l'extrémité de sortie du conduit, afin de récupérer la poudre chaude.

6. Appareil selon la revendication 5, dans lequel la trémie est isolée thermiquement.

7. Appareil selon la revendication 5 ou la revendication 6, comportant en outre un refroidisseur (17) placé en aval de ladite trémie.

8. Appareil selon la revendication 7, dans lequel le refroidisseur (17) comprend un tube sensiblement vertical le long duquel passe la poudre du haut vers le bas, et une enveloppe extérieure de circulation d'eau à travers laquelle circule un réfrigérant lors du fonctionnement.

9. Appareil selon la revendication 7 ou la revendication 8, dans lequel le tube de stérilisation et le tube de refroidissement sont disposés verticalement et adjacents l'un à l'autre, la trémie (16) étant située au dessus du tube de refroidissement (17) et un moyen élévateur (15) étant prévu pour transporter la poudre de la base du tube de stérilisation jusqu'à la trémie.

10. Procédé de stérilisation de poudres fines comprenant les étapes suivantes :
a) chauffage d'un conduit de stérilisation jusqu'à une température d'environ 500°C ;
b) introduction d'une poudre fine à une extrémité du conduit et enlèvement de la poudre fine à l'autre extrémité du conduit, la durée de séjour dans le conduit étant suffisant pour chauffer la poudre à une température supérieure à 120°C ;
c) introduction d'air dans le conduit à une vitesse de 30 à 60 litres par minute.

11. Procédé de stérilisation de poudres fines selon la revendication 10 comprenant en outre les étapes suivantes :
d) maintien de la poudre fine à une température élevée pendant au moins 15 minutes à la sortie du conduit de stérilisation pour qu'elle puisse s'imprégner ;
e) refroidissement de la poudre fine en vue de son évacuation et de son traitement ultérieur.
